(19) Europäisches Patentamt
European Patent Office
Office européen des brevets

(11) Veröffentlichungsnummer: **0 298 380 B1**

(12) **EUROPÄISCHE PATENTSCHRIFT**

(45) Veröffentlichungstag der Patentschrift: **29.09.93**

(21) Anmeldenummer: **88110516.7**

(22) Anmeldetag: **01.07.88**

(51) Int. Cl.⁵: **C07D 213/48**, C07D 309/32, C07D 335/02, C07C 47/24, C07D 239/26, C07D 241/12, C07D 333/22, C07D 307/46, C07D 261/08, C07D 263/60, C07D 277/24

(54) **Verfahren zur Herstellung von alpha-beta-substituierten Acroleinen.**

(30) Priorität: **10.07.87 DE 3722886**

(43) Veröffentlichungstag der Anmeldung:
**11.01.89 Patentblatt 89/02**

(45) Bekanntmachung des Hinweises auf die Patenterteilung:
**29.09.93 Patentblatt 93/39**

(84) Benannte Vertragsstaaten:
**BE CH DE ES FR GB IT LI NL**

(56) Entgegenhaltungen:
**CH-A- 563 383**
**CH-A- 609 053**
**DE-A- 2 516 623**
**DE-B- 2 614 797**
**US-A- 2 527 714**

(73) Patentinhaber: **BASF Aktiengesellschaft**
**Carl-Bosch-Strasse 38**
**D-67063 Ludwigshafen(DE)**

(72) Erfinder: **Goetz, Norbert, Dr.**
**Schoefferstrasse 25**
**D-6520 Worms 1(DE)**
Erfinder: **Karbach, Stefan, Dr.**
**Sperlinggasse 3**
**D-6700 Ludwigshafen(DE)**
Erfinder: **Recker, Hans-Gert, Dr.**
**Lisztstrasse 117**
**D-6700 Ludwigshafen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 298 380 B1

CHEMICAL ABSTRACTS, Band 49, Nr 20, 25. Oktober 1955, Spalte 13952h - 13953c, Columbus, Ohio, USA ; A. ETIENNE et al.:"1,2-diphenyl-1,3-butadiene and its addition products with maleic anhydride and naphthoquinone"

CHEMICAL ABSTRACTS, Band 49, Nr. 8, 25. April 1985, Spalte 5394d - 5396d, Columbus, Ohio, USA ; K. ALDER et al.: "Diene syntheses. XLII. Diene syntheses with unsymetrical addends. Preparation of certain 1,2-substituted dienes and their applicability in diene syntheses"

HOUBEN-WEYL/METHODEN DER ORGANISCHEN CHEMIE, 4. Auflage, Band VII, Teil 1, 1954, Seiten 76-85, Georg Thieme Verlag, Stuttgart, DE

## Beschreibung

Die vorliegende Erfindung betrifft ein Verfahren zur Herstellung von $\alpha,\beta$-substituierten Acroleinen der allgemeinen Formel I

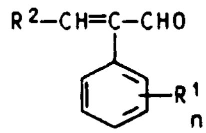

I

in der der Rest $R^1$ für die $C_1$- bis $C_4$-Alkoxy-, Phenoxy-, Halogen-, $C_1$- bis $C_4$-Halogenalkyl-, $C_1$-$C_4$-Halogenalkoxy-, $C_1$- bis $C_4$-Halogenalkylthiyl- oder Nitro-Gruppe steht, n eine ganze Zahl von 1 bis 5 bedeutet, wobei bei n größer 1 die Reste $R^1$ gleich oder verschieden sein können und im Falle einer Substitution durch Nitrogruppen der aromatische Ring nicht mehr als 3 Nitrogruppen trägt und worin $R^2$ für einen $C_1$- bis $C_{10}$-Alkyl-, $C_5$- bis $C_6$-Cycloalkyl-, einen unsubstituierten oder substituierten Aryl- oder Heteroarylrest mit 1 bis 3 Heteroatomen, ausgewählt aus Stickstoff, Sauerstoff und Schwefel, oder einen nichtaromatischen heterocyclischen Rest mit 5 oder 6 Ringgliedern und 1 bis 3 Heteroatomen, ausgewählt aus Stickstoff, Sauerstoff und Schwefel, steht.

Es ist bekannt, daß gekreuzte Aldolkondensationen in Abhängigkeit von der Struktur und Reaktivität der eingesetzten Aldehyde häufig zu Produktgemischen unterschiedlicher Zusammensetzung bei oftmals geringen Ausbeuten führen (Houben-Weyl, Methoden der organischen Chemie, Bd. VII/1, 76 ff).

So erhält Alder (Ann. d. Chemie, 586, 110 (1954)) $\alpha$-Phenylzimtaldehyd aus Phenylacetaldehyd und Benzaldehyd unter basischen Kondensationsbedingungen in nur mäßigen Ausbeuten von 69 %.

Bei der Umsetzung von Phenylacetaldehyd mit Isovaleraldehyd unter basischen Bedingungen können nach fraktionierter Destillation nur äußerst geringe Ausbeuten (16,5 %) des gewünschten Aldolproduktes 5-Methyl-2-Phenyl-2-Hexenal isoliert werden (DE-A 19 21 560). Die notwendige Aufreinigung und Trennung derartiger Produktgemische ist offensichtlich problematisch und äußerst kostenaufwendig; gemischte Aldolkondensationen sind daher technisch nur von geringem Interesse (A. T. Nielsen, W. J. Houlihar, Org. Reactions 16, Seiten 15 ff (1968), Houben-Weyl VII/1, Seite 79).

Durch Untersuchungen von H. Stobbe und A. Lippold (J. f. prakt. Chemie 90, 277 ff (1912)) ist weiterhin die hohe Tendenz des Phenylacetaldehydes zur Selbstkondensation in Gegenwart von Säuren oder Basen bekannt. Die Beobachtungen von W. Treibs und K. Krumbholz (Chem. Ber. 85, 1116 ff (1952)) zur spontanen Polymerisation des Phenylacetaldehydes unter sauren oder basischen Bedingungen bestätigen die Probleme bei der Handhabung dieser Substanzklasse.

Der Erfindung lag nun die Aufgabe zugrunde, speziell substituierte Acroleine mit einem Phenylrest in 2-Stellung herzustellen, wobei der Phenylrest zusätzlich substituiert sein sollte, insbesondere durch elektronenziehende Reste. Der Syntheseweg sollte sich durch leicht zugängliche Ausgangsmaterialien, gute Ausbeuten, hohe Chemoselektivität, einfache Produktisolierung und damit problemlose Durchführbarkeit im technischen Anwendungsbereich auszeichnen.

Demgemäß wurde ein Verfahren zur Herstellung von $\alpha,\beta$-substituierten Acroleinen der allgemeinen Formel I

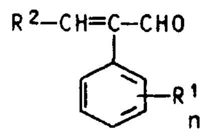

I

worin der Rest $R^1$ für die Alkoxy-, Phenoxy-, Halogen-, Halogenalkyl-, Halogenalkoxy-, Halogenalkylthiyl- oder Nitro-Gruppe steht, n eine ganze Zahl von 1 bis 5 bedeutet, wobei bei n größer 1 die Reste $R^1$ gleich oder verschieden sein können und im Falle einer Substitution durch Nitrogruppen der aromatische Ring nicht mehr als 3 Nitrogruppen trägt und worin $R^2$ für einen Alkyl-, Cycloalkyl-, einen unsubstituierten oder substituierten Aryl- oder Heteroaryl- oder einen nichtaromatischen heterocyclischen Rest steht, gefunden, welches dadurch gekennzeichnet ist, daß man einen Phenylacetaldehyd der allgemeinen Formel II

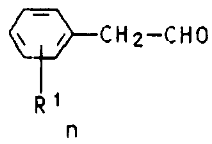

II

mit einem Aldehyd der allgemeinen Formel III

[$R^2$-CHO]    III

in Gegenwart einer Base und eines Lösungsmittels umsetzt.

Weiterhin wurden die teilweise neuen $\alpha,\beta$-substituierten Acroleine der allgemeinen Formel Ia

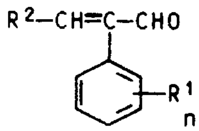

Ia

gefunden, in der $R^1$ für die Halogen-, $C_1$-$C_4$-Alkoxy-, Phenoxy-, $C_1$-$C_4$-Halogenalkyl-, $C_1$-$C_4$-Halogenalkoxy- oder Nitro-Gruppe steht, n eine ganze Zahl 1 bis 5 ist, wobei bei n größer 1 die Reste $R^1$ gleich oder verschieden sein können und im Falle einer Substitution durch Nitrogruppen der aromatische Ring nicht mehr als 3 Nitrogruppen trägt und $R^2$ einen heteroaromatischen oder nichtaromatischen heterocyclischen Rest mit 1 bis 3 Stickstoff-, Sauerstoff- und/oder Schwefelatomen darstellt.

Der Erfolg des erfindungsgemäßen Verfahrens ist überraschend, da bekanntermaßen die Einführung insbesondere elektronenziehender Substituenten $R^1$ in Verbindungen der allgemeinen Formel II die Reaktivität der benzylischen $CH_2$-Gruppe drastisch erhöht, so daß zu erwarten war, daß die substituierten Phenylacetaldehyde eine starke Tendenz zur Selbstkondensation zeigen würden.

Beispielsweise führt die gekreuzte Aldolkondensation von 2-Ethoxycarbonylmethoxyphenylacetaldehyd mit Benzaldehyd nur zu etwa 30% zum gewünschten Kondensationsprodukt $\alpha$-(-2-Methoxycarbonylmethoxyphenyl)-Zimtaldehyd (DE-A 25 16 623).

Als Ausgangsstoffe II werden bevorzugt Verbindungen mit elektronenziehenden Substituenten, wie Halogenatomen, z.B. Fluor, Chlor oder Brom-Halogenalkylgruppen mit z.B. 1 bis 10, insbesondere 1 bis 4 C-Atomen und 1 bis 3 Halogenatomen, wie Brom, Chlor oder Fluor, Halogenalkoxygruppen oder Halogenalkylthiylgruppen mit 1 bis 4 C-Atomen und mit 1 bis 3 der genannten Halogenatome, verwendet. $R^1$ kann darüber hinaus für $C_1$-bis $C_4$-Alkoxy-, Phenoxy- oder Nitrogruppen stehen. Bevorzugt trägt der Phenylkern 1, 2 oder 3 Substituenten. Beispielsweise seien folgende Verbindungen aufgeführt:
2-Fluor-, 3-Fluor-, 4-Fluor-, 2-Chlor-, 4-Chlor-, 4-Brom-, 2,4-Dichlor-, 2,4-Difluor-, 3,4-Dichlor-, 2,6-Dichlor-, 2,6-Difluor-, 2-Chlor-6-fluor-, 2-, 3- oder 4-Trifluormethyl- oder 4-Trifluormethoxiphenylacetaldehyd.

Als Ausgangsstoffe III kommen Verbindungen in Betracht, in denen $R^2$ einen verzweigten oder unverzweigten Alkylrest, z.B. mit 1 bis 10, insbesondere 1 bis 4 Kohlenstoffatomen, einen Cycloalkylrest mit 5- oder 6 Kohlenstoffatomen, einen substituierten oder unsubstituierten Arylrest, z.B. einen Phenyl-, p-Biphenyl-, Naphthylrest, wobei als Substituenten Halogenatome, wie Chlor, Brom und/oder Fluor, $C_1$-$C_4$-Alkoxy-, $C_1$-$C_4$-Alkyl-, Phenoxy-, Nitro-, $C_1$-$C_4$-Halogenalkyl- oder Phenylsulfonylgruppen zu nennen sind, einen Heteroarylrest, insbesondere mit 1 bis 3 Heteroatomen, wie Sauerstoff, Schwefel und vorzugsweise Stickstoff, besonders bevorzugt mit 1 oder 2 Stickstoff-, Sauerstoff- und/oder Schwefelatomen, oder einen gesättigten oder ungesättigten heterocyclischen Rest mit 5 oder 6 Ringgliedern und mit 1 bis 3, insbesondere einem der genannten Heteroatome, darstellt. Beispielsweise seien folgende Reste $R^2$ aufgeführt: Methyl, Ethyl, Propyl, Isopropyl, Butyl, tert.-Butyl, Cyclopentyl, Cyclohexyl, 1-Naphthyl, 2-Naphthyl, p-Biphenyl, Phenyl, 2-Chlorphenyl, 3-Chlorphenyl, 4-Chlorphenyl, 2-Fluorphenyl, 3-Fluorphenyl, 2-Fluor-6-Chlorphenyl, 4-Fluorphenyl, 4-Bromphenyl, 2,4-Dichlorphenyl, 3,4-Dichlorphenyl, 3,5-Dichlorphenyl, 2,6-Dichlorphenyl, 3-Chlor-4-methylphenyl, 2-Methoxyphenyl, 3-Methoxyphenyl, 2,4-Dimethoxyphenyl, 3,4-Dimethoxyphenyl, 4-Methoxyphenyl, 4-Ethoxyphenyl, 4-tert.-Butoxyphenyl, 4-Methylphenyl, 4-Ethylphenyl, 4-Isopropylphenyl, 4-tert.-Butylphenyl, 4-Phenoxyphenyl, 3-Phenoxyphenyl, 3-Nitrophenyl, 4-Nitrophenyl, 4-Nitro-2-Chlorphenyl, 3-Trifluormethylphenyl, 4-Trifluormethylphenyl oder 4-Phenylsulfonylphenyl, 2-, 3- oder 4-Pyridyl, 2-, 3- oder 4-Pyridazinyl, 2-Pyrimidyl, 4-Pyrimidyl, 2-Thienyl, 3-Thienyl, 2-Furanyl, 3-Pyranyl, 2-, 3- oder 4-Pyranyl, 2-, 3- oder 4-Thiopyranyl, 2-, 4- oder 5-Oxazolyl, 2-Thiazolyl, 3-, 4- oder 5-Isoxazolyl, 3-Piperidyl.

Wichtige erfindungsgemäße Verbindungen I sind insbesondere die in der Tabelle zur allgemeinen Arbeitsvorschrift aufgeführten Verbindungen sowie u.a. die folgenden Propenale:
2-(3,4-Dichlorphenyl)-3-(3-pyridyl)-propenal
2-(2,6-Dichlorphenyl)-3-(3-pyridyl)-propenal
2-(2-Trifluormethylphenyl)-3-(3-pyridyl)-propenal
2-(3-Trifluormethylphenyl)-3-(3-pyridyl)-propenal
2-(4-Trifluormethylphenyl)-3-(3-pyridyl)-propenal
2-(4-Trifluormethoxyphenyl)-3-(3-pyridyl)-propenal
2-(2-Methyl-4-fluorphenyl)-3-(3-pyridyl)-propenal
2-(2-Chlorphenyl)-3-(3-pyridyl)-propenal
2-(2-Fluorphenyl)-3-(3-pyridyl)-propenal
2-(3-Fluorphenyl)-3-(3-pyridyl)-propenal
2-(2,6-Difluorphenyl)-3-(3-pyridyl)-propenal
2-(2-Chlor-6-fluorphenyl)-3-(3-pyridyl)-propenal
2-(4-Bromphenyl)-3-(3-pyridyl)-propenal
2-(4-Chlorphenyl)-3-(2-pyridyl)-propenal
2-(4-Fluorphenyl)-3-(2-pyridyl)-propenal
2-(4-Chlorphenyl)-3-(4-pyridyl)-propenal
2-(4-Fluorphenyl)-3-(4-pyridyl)-propenal
2-(4-Fluorphenyl)-3-(4-thiopyranyl)-propenal
2-(4-Fluorphenyl)-3-(2-thiopyranyl)-propenal
2-(4-Fluorphenyl)-3-(2-furanyl)-propenal
2-(4-Fluorphenyl)-3-(2-thienyl)-propenal
2-(4-Fluorphenyl)-3-(3-thienyl)-propenal
2-(4-Fluorphenyl)-3-(3-isoxazolyl)-propenal
2-(4-Fluorphenyl)-3-(4-isoxazolyl)-propenal
2-(4-Fluorphenyl)-3-(5-isoxazolyl)-propenal
2-(4-Fluorphenyl)-3-(2-oxazolyl)-propenal
2-(4-Fluorphenyl)-3-(4-oxazolyl)-propenal
2-(4-Fluorphenyl)-3-(5-oxazolyl)-propenal
2-(4-Fluorphenyl)-3-(2-thiazolyl)-propenal
2-(4-Fluorphenyl)-3-(2-pyrimidinyl)-propenal
2-(4-Fluorphenyl)-3-(4-pyrimidinyl)-propenal
2-(4-Fluorphenyl)-3-(3-pyridazinyl)-propenal
2-(4-Fluorphenyl)-3-(4-pyridazinyl)-propenal
2-(4-Fluorphenyl)-3-(3-pyrazinyl)-propenal.

Die Umsetzung der Ausgangsstoffe II und III wird in Gegenwart von für Aldolkondensationen üblichen Basen durchgeführt.

Geeignete Basen sind beispielsweise Alkali- und Erdalkalihydroxide, wie z.B. Natrium-, Kalium-, Lithium-, Barium- und Calciumhydroxid; Alkali- und Erdalkalicarbonate wie z. B. Natrium-, Kalium-, Lithium- und Calciumcarbonat; ferner Alkoholate wie z. B. Natrium- oder Kaliummethylat, -ethylat, -propylat, -isopropylat, -n-butanolat, -iso-butanolat, tert.-butanolat, -cyclohexanolat sowie primäre und sekundäre Amine wie z. B. Piperidin, Pyrrolidin, Diisopropylamin.

Die Basenmenge liegt im allgemeinen bei 0,01 bis 1 mol, insbesondere 0,01 bis 0,5 mol pro Mol Ausgangsstoff II.

Das Verhältnis der Ausgangsstoffe II zu III kann 1 bis 0,1, insbesondere 1 bis 0,5 betragen. Häufig sind äquimolare Mengen vorteilhaft, um Nebenreaktionen zu vermeiden.

Die Umsetzung von II mit III kann in Abwesenheit oder vorteilhaft in Gegenwart eines unter den Reaktionsbedingungen inerten Lösungsmittels vorgenommen werden. Geeignete Lösungsmittel sind z.B. Ether, wie Methyl-tert.-Butylether, Tetrahydrofuran, Dimethoxyethan, Dioxan, Amide, wie z.B. Dimethylformamid, Dimethylacetamid und N-Methylpyrrolidon, Harnstoffderivate, wie z. B. DMPU oder DMEU (DMPU: N,N'-Dimethylpropylenharnstoff, DMEU: N,N'-Dimethylethylenharnstoff). Besonders geeignet sind Kohlenwasserstoffe oder halogenierte Kohlenwasserstoffe, wie z.B. Pentan, Hexan, Heptan, Cyclohexan, Toluol, Xylol, Methylenchlorid, Chloroform, Tetrachlorkohlenstoff, Dichlorethan oder Chlorbenzol und insbesondere niedermolekulare Alkohole, wie z.B. Methanol, Ethanol, Isopropanol, n-Propanol, n-Butanol, iso-Butanol, tert.-Butanol oder Cyclohexanol. Die besten Ausbeuten werden mit Methanol oder Methanol/Wasser-Gemischen als Lösungsmittel erzielt.

Die Menge des Lösungsmittels liegt im allgemeinen bei 100 - 300 ml/mol.

Die Umsetzung kann bei Temperaturen von -20 °C bis +120 °C, bevorzugt bei -5 °C bis +60 °C, besonders bevorzugt bei 0 °C bis 40 °C drucklos oder unter Druck, kontinuierlich oder diskontinuierlich nach den dafür üblichen Techniken durchgeführt werden.

Zweckmäßigerweise wird die Umsetzung so durchgeführt, daß man den Aldehyd III im Lösungsmittel zusammen mit der gewünschten Base vorlegt und den Phenylacetaldehyd II zudosiert oder die Base mit dem Lösungsmittel bei Reaktionstemperatur vorlegt und die geeignete Mischung aus dem Phenylacetaldehyd (Aldehyd II) und dem Aldehyd III zugibt.

Nach Beendigung der Umsetzung kann das Endprodukt auf übliche Weise, z.B. durch Extraktion mit geeigneten organischen Lösungsmitteln, z.B. chlorierten Kohlenwasserstoffen, Kohlenwasserstoffen, Estern, Ethern oder besonders bevorzugt durch direkte Kristallisation aus der Reaktionsmischung isoliert werden.

Die Produkte der Formel I können als E- oder Z-Isomere entstehen. Die Isomerengemische lassen sich in üblicher Weise, beipielsweise aufgrund ihrer unterschiedlichen Löslichkeit oder durch Säulenchromatographie trennen und in reiner Form isolieren. Bei der Synthese der unten aufgeführten Verbindungen überwiegt in den meisten Fällen in dem erhaltenen Isomerengemisch das E-Isomere mit einem Anteil von 90 bis 99%.

Die nach dem erfindungsgemäßen Verfahren zugänglichen Acroleine I dienen zur Herstellung von Hydroxymethyloxiranen, welche wertvolle Zwischenprodukte zur Synthese antimykotisch und fungizid wirksamer Azolylmethyloxirane darstellen. Derartige Azolylmethyloxirane sind in der EP-A 94 564 beschrieben. Die Umsetzung der Acroleine I zu Hydroxymethyloxiranen kann entsprechend der in der älteren Patentanmeldung DE-A 36 01 927 angegebenen Arbeitsweise, durch Epoxidierung zu Formyloxiranen und anschließende Reduktion, vorgenommen werden.

Beispiel

Allgemeine Arbeitsvorschrift

0,1 mol NaOH wurden in 250 ml Methanol gelöst und unter Eiskühlung 1.1 mol Aldehyd III hinzugetropft. Anschließend wurde bei 20-30 °C innerhalb von ca. 4 bis 5 Stunden 1 mol Phenylacetaldehyd II hinzugetropft. Nach etwa 30 Minuten wurde dann mit 10%iger $H_2SO_4$ auf pH 7 eingestellt. Nach Eindampfen im Vakuum wurde das verbleibende Reaktionsgemisch fraktioniert destilliert.

Ausgangsstoffe und physikalische Daten der Produkte sind der nachfolgenden Tabelle zu entnehmen.

Tabelle

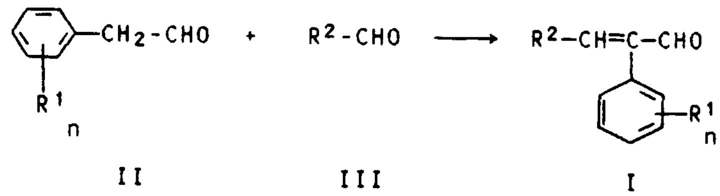

| Bsp. | $R^1_n$ | $R^2$ | Ausbeute I % | Siedepunkt I mbar/°C |
|---|---|---|---|---|
| 1 | H | 3-Pyridyl | 86 | 0,6 /160 (Fp 94°C) |
| 2 | 4-Fluor | 3-Pyridyl | 85 | 0,6 /153-156 |
| 3 | 2,4-Dichlor | 3-Pyridyl | 91 | 0,5 /167 |

| Bsp. | $R^1_n$ | $R^2$ | Ausbeute I % | Siedepunkt I mbar/°C |
|---|---|---|---|---|
| 4 | 2,4-Difluor | 3-Pyridyl | 85 | 0,5 /150 |
| 5 | 3,4-Difluor | 3-Pyridyl | 87 | 0,5 /150 |
| 6 | 4-Chlor | 3-Pyridyl | 91 | 0,5 /163 |
| 7 | H | 4-Pyridyl | 81 | 0,35/160 |
| 8 | 4-Fluor | 4-Pyranyl | 94 | 0,3 /142 |
| 9 | 4-Chlor | 4-Pyranyl | 94 | 0,3 /148 |
| 10 | 4-Fluor | 3-Pyranyl | 96 | 0,3 /130 |
| 11 | 4-Fluor | 2-Pyranyl | 92 | 0,3 /138 |
| 12 | 4-Fluor | 3-Thiopyranyl | 91 | 0,3 /142 |
| 13 | 4-Fluor | n-Butyl | 81 | 0,1 / 89- 91 |
| 14 | 4-Fluor | 2-Chlorphenyl | 93 | 89 -92 (Fp) |

## Patentansprüche

**1.** Verfahren zur Herstellung von $\alpha,\beta$-substituierten Acroleinen der allgemeinen Formel I

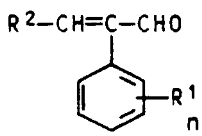

I,

in der der Rest $R^1$ für die $C_1$- bis $C_4$-Alkoxy-, Phenoxy-, Halogen-, $C_1$- bis $C_4$-Halogenalkyl-, $C_1$- bis $C_4$-Halogenalkoxy-, $C_1$- bis $C_4$-Halogenalkylthiyl- oder Nitro-Gruppe steht, n eine ganze Zahl von 1 bis 5 bedeutet, wobei bei n größer 1 die Reste $R^1$ gleich oder verschieden sein können und im Falle einer Substitution durch Nitrogruppen der aromatische Ring nicht mehr als 3 Nitrogruppen trägt und worin $R^2$ für einen $C_1$- bis $C_{10}$-Alkyl-, $C_5$- bis $C_6$-Cycloalkyl-, einen unsubstituierten oder einen durch Halogenatome, $C_1$-$C_4$-Alkoxy-, $C_1$-$C_4$-Alkyl-, Phenoxy-, Nitro-, $C_1$-$C_4$-Halogenalkyl oder Phenylsulfonyl- substituierten Aryl-rest oder einen Heteroarylrest mit 1 bis 3 Heteroatomen, ausgewählt aus Stickstoff, Sauerstoff und Schwefel, oder einen nichtaromatischen heterocyclischen Rest mit 5 oder 6 Ringgliedern und 1 bis 3 Heteroatomen, ausgewählt aus Stickstoff, Sauerstoff und Schwefel, steht, dadurch gekennzeichnet, daß man einen Phenylacetaldehyd der allgemeinen Formel II

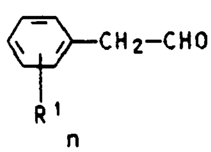

II

mit einem Aldehyd der allgemeinen Formel III

[$R^2$-CHO] III

in Gegenwart einer Base und eines Lösungsmittels umsetzt.

**2.** Verfahren nach Anspruch 1, dadurch gekennzeichnet, daß $R^2$ einen Arylrest oder einen Heteroarylrest mit 1 oder 2 Stickstoff-, Sauerstoff- und/oder Schwefelatomen bedeutet.

**3.** Verfahren nach Anspruch 1, dadurch gekennzeichnet, daß n einen Wert von 1 bis 3 hat.

4. Verfahren nach Anspruch 1, dadurch gekennzeichnet, daß $R^1$ Fluor, Chlor, Brom oder einen $C_1$-$C_4$-Halogenalkylrest darstellt.

5. Verfahren nach Anspruch 1, dadurch gekennzeichnet, daß man als Base primäre oder sekundäre Amine verwendet.

6. Verfahren nach Anspruch 1, dadurch gekennzeichnet, daß man die Umsetzung in niedermolekularen Alkoholen als Lösungsmittel vornimmt.

7. Verfahren nach Anspruch 6, dadurch gekennzeichnet, daß man die Umsetzung in Methanol oder Methanol/Wasser-Gemischen vornimmt.

**Claims**

1. A process for preparing an $\alpha,\beta$-substituted acrolein of the formula I

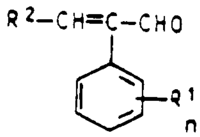

I.

where $R^1$ is $C_1$-$C_4$-alkoxy, phenoxy, halogen, $C_1$-$C_4$-haloalkyl, $C_1$-$C_4$-haloalkoxy, $C_1$-$C_4$-haloalkylthiyl or nitro, n is an integer from 1 to 5, in the case of n being greater than 1 the $R^1$s being identical or different and in the event of nitro substitution the aromatic ring carrying not more than 3 nitro groups, and where $R^2$ is $C_1$-$C_{10}$-alkyl, $C_5$- or $C_6$-cycloalkyl, unsubstituted or halogen-, $C_1$-$C_4$-alkoxy-, $C_1$-$C_4$-alkyl-, phenoxy-, nitro-, $C_1$-$C_4$-haloalkyl- or phenylsulfonyl-substituted aryl or hetaryl containing 1 to 3 hetero atoms selected from the group consisting of nitrogen, oxygen and sulfur, or a nonaromatic heterocyclic radical containing 5 or 6 ring members and 1 to 3 hetero atoms selected from the group consisting of nitrogen, oxygen and sulfur, which comprises reacting a phenylacetaldehyde of the formula II

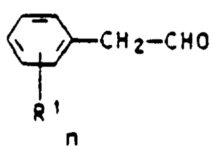

II

with an aldehyde of the formula III

[$R^2$-CHO] III

in the presence of a base and a solvent.

2. A process as claimed in claim 1, wherein $R^2$ is aryl or hetaryl containing 1 or 2 nitrogen, oxygen and/or sulfur atoms.

3. A process as claimed in claim 1, wherein n is from 1 to 3.

4. A process as claimed in claim 1, wherein $R^1$ is fluorine, chlorine, bromine or $C_1$-$C_4$-haloalkyl.

5. A process as claimed in claim 1, wherein the base used is a primary or secondary amine.

6. A process as claimed in claim 1, wherein the reaction is carried out in a low molecular weight alcohol as a solvent.

7. A process as claimed in claim 6, wherein the reaction is carried out in methanol or a methanol/water mixture.

## Revendications

1. Procédé de préparation d'acroléines $\alpha,\beta$-substituées de formule générale I

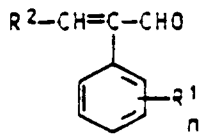

I.

dans laquelle le reste $R^1$ est mis pour un groupement alcoxy en $C_1$-$C_4$, phénoxy, halogéno, halogénoalkyle en $C_1$-$C_4$, halogénoalcoxy en $C_1$-$C_4$, halogénoalkylthioyle en $C_1$-$C_4$ ou nitro, n est un nombre entier de 1 à 5, les restes $R^1$, quand n est supérieur à 1, pouvant être identiques ou différents et, en cas de substitution par des groupements nitro, le noyau aromatique ne portant pas plus de 3 groupements nitro, et dans laquelle $R^2$ est mis pour un reste alkyle en $C_1$-$C_{10}$, cycloalkyle en $C_5$ ou $C_6$, pour un reste aryle non substitué ou substitué par des atomes d'halogène, des groupements alcoxy en $C_1$-$C_4$, alkyle en $C_1$-$C_4$, phénoxy, nitro, halogénoalkyle en $C_1$-$C_4$ ou phénylsulfonyle, pour un reste hétéroaryle renfermant 1 à 3 hétéroatomes choisis parmi l'azote, l'oxygène et le soufre, ou pour un reste hétérocyclique non aromatique à 5 ou 6 termes cycliques et à 1 à 3 hétéroatomes choisis parmi l'azote, l'oxygène et le soufre, caractérisé en ce qu'on fait réagir un phénylacétaldéhyde de formule générale II

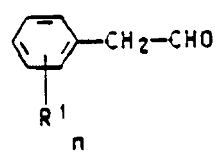

II

avec un aldéhyde de formule générale III

[$R^2$-CHO] III

en présence d'une base et d'un solvant.

2. Procédé selon la revendication 1, caractérisé en ce que $R^2$ représente un reste aryle ou un reste hétéroaryle renfermant 1 ou 2 atomes d'azote, d'oxygène et/ou de soufre.

3. Procédé selon la revendication 1, caractérisé en ce que n a une valeur de 1 à 3.

4. Procédé selon la revendication 1, caractérisé en ce que $R^1$ représente un atome de fluor, de chlore, de brome ou un reste halogénoalkyle en $C_1$-$C_4$.

5. Procédé selon la revendication 1, caractérisé en ce qu'on utilise, comme bases, des amines primaires ou secondaires.

6. Procédé selon la revendication 1, caractérisé en ce qu'on conduit la réaction dans des alcools inférieurs servant de solvant.

7. Procédé selon la revendication 6, caractérisé en ce qu'on conduit la réaction dans du méthanol ou dans des mélanges méthanol/eau.